(19) Europäisches Patentamt
European Patent Office
Office européen des brevets

(11) Veröffentlichungsnummer: **0 374 622 A2**

(12) # EUROPÄISCHE PATENTANMELDUNG

(21) Anmeldenummer: **89122654.0**

(51) Int. Cl.5: **C07D 253/075, A01N 43/707**

(22) Anmeldetag: **08.12.89**

(30) Priorität: **21.12.88 DE 3842932**

(43) Veröffentlichungstag der Anmeldung:
**27.06.90 Patentblatt 90/26**

(84) Benannte Vertragsstaaten:
**BE CH DE FR GB IT LI NL**

(71) Anmelder: **BAYER AG**
**D-5090 Leverkusen 1 Bayerwerk(DE)**

(72) Erfinder: **Kranz, Eckart, Dr.**
**Am Acker 9**
**D-5600 Wuppertal 1(DE)**
Erfinder: **Santel, Hans-Joachim, Dr.**
**Gruenstrasse 9a**
**D-5090 Leverkusen(DE)**
Erfinder: **Schmidt, Robert R., Dr.**
**Im Waldwinkel 110**
**D-5060 Bergisch-Gladbach 2(DE)**

(54) **3,4-Bis-(methylamino)-6-(2,3-dimethyl-2-butyl)-1,2,4-triazin-5(4H)-on, Verfahren zu seiner Herstellung und seine Verwendung als Herbizid.**

(57) Die Erfindung betrifft ein neues Triazinon-Derivat der Formel (I),

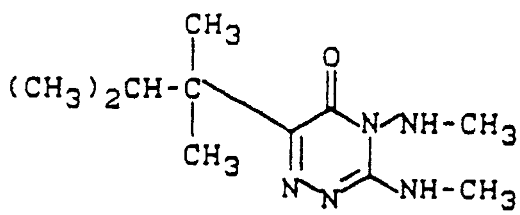

(I),

ein Verfahren zu seiner Herstellung sowie seine Verwendung als Herbizid.

EP 0 374 622 A2

# 3,4-Bis-(methylamino)-6-(2,3-dimethyl-2-butyl)-1,2,4-triazin-5(4H)-on, Verfahren zu seiner Herstellung und seine Verwendung als Herbizid

Die vorliegende Erfindung betrifft ein neues Triazinon-Derivat, ein Verfahren zu seiner Herstellung sowie seine Verwendung als Herbizid.

Es ist bereits bekannt, daß bestimmte 3,4-Bis-(methylamino)-1,2,4-triazin-5(4H)-on-Derivate, wie beispielsweise das 3,4-Bis-(methylamino)-6-tert-butyl-1,2,4-triazin-5(4H)-on, herbizide Eigenschaften besitzen (vgl. EP-A-0 114 783 und EP-A-0 150 677). Ferner ist die Verbindung 4-Amino-3-dimethylamino-6-(2,3-dimethyl-2-butyl)-1,2,4-triazin-5(4H)-on als Herbizid vorgeschlagen worden (vgl. DE-A-32 40 308).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Es wurde das neue 3,4-Bis-(methylamino)-6-(2,3-dimethyl-2-butyl)-1,2,4-triazin-5(4H)-on der Formel (I)

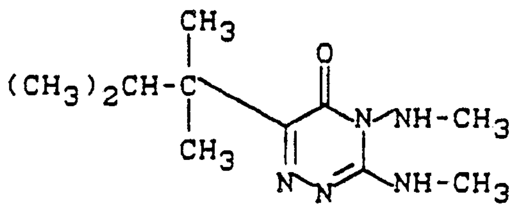

(I)

gefunden.

Weiterhin wurde gefunden, daß man das neue 3,4-Bis-(methylamino)-6-(2,3-dimethyl-2-butyl )-1,2,4-triazin-5-(4H)-on der Formel (I) erhält, wenn man ein 3-Alkylthio-4-methylamino-6-(2,3-dimethyl-2-butyl )-1,2,4-triazin-5-(4H)-on der Formel (II)

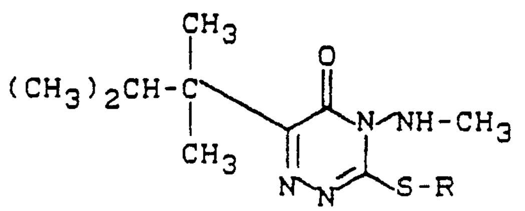

(II)

in welcher

R für Alkyl steht,

gegebenenfalls in Gegenwart eines Verdünnungsmittels und gegebenenfalls in Gegenwart einer niederen aliphatischen Carbonsäure mit Methylamin umsetzt.

Schließlich wurde gefunden, daß das neue 3,4-Bis-(methylamino)-6-(2,3-dimethyl-2-butyl)-1,2,4-triazin-5-(4H)-on der Formel (I) herbizide, insbesondere selektiv herbizide Eigenschaften besitzt.

Überraschenderweise zeigt das erfindungsgemäße 3,4-Bis-(methylamino)-6-(2,3-dimethyl-2-butyl)-1,2,4-triazin-5-(4H)-on der Formel (I) bei einer vergleichbaren herbiziden Wirksamkeit gegenüber wichtigen Problemunkräutern gleichzeitig eine deutlich verbesserte Verträglichkeit gegenüber wichtigen Kulturpflanzen, wie insbesondere Mais, Weizen und Gerste, beispielsweise im Vergleich zu dem 3,4-Bis-(methylamino)-6-tert-butyl-1,2,4-triazin-5-(4H)-on, welches chemisch und wirkungsmäßig eine sehr naheliegende Verbindung ist.

Verwendet man beispielsweise 6-(2,3-Dimethyl-2-butyl)-4-methylamino-3-methylthio-1,2,4-triazin-5(4H)-on und Methylamin als Ausgangsprodukte, so läßt sich der Reaktionsablauf des erfindungsgemäßen Verfahrens durch das folgende Formelschema wiedergeben:

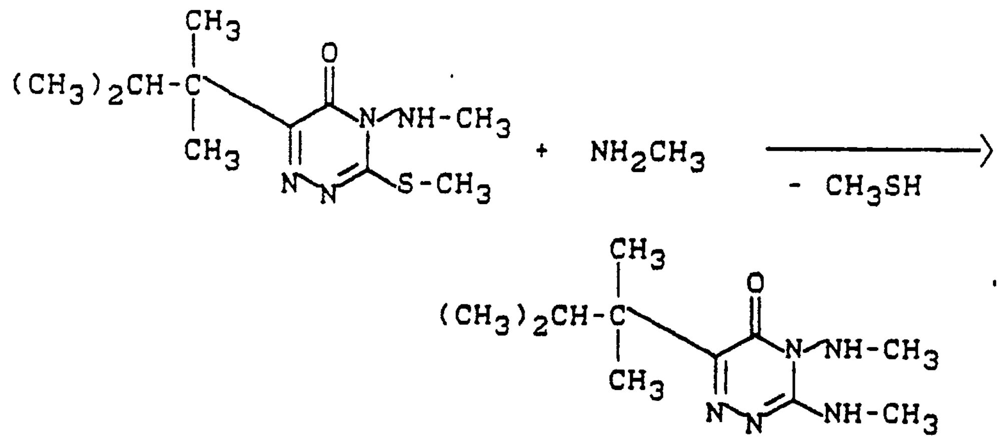


Die zur Durchführung des erfindungsgemäßen Verfahrens als Ausgangsstoffe benötigten 3-Alkylthio-4-methylamino-6-(2,3-dimethyl-2-butyl)-1,2,4-triazin-5(4H)-one sind durch die Formel (II) allgemein definiert. In dieser Formel (II) steht R vorzugsweise für Alkyl mit 1 bis 4 Kohlenstoffatomen, insbesondere für Methyl oder Ethyl.

Die Verbindungen der Formel (II) besitzen selbst auch herbizide Eigenschaften.

Die 3-Alkylthio-4-methylamino-6-(2,3-dimethyl-2-butyl)-1,2,4-triazin-5(4H)-one der Formel (II) sind noch nicht bekannt. Man erhält sie jedoch in prinzipiell bekannter Weise aus den 4-Amino-Verbindungen der Formel (III),

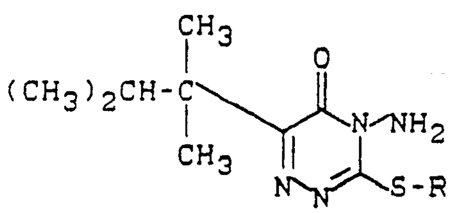
(III)

in welcher
R die oben angegebene Bedeutung hat,
indem man diese mit einem Alkylierungsmittel, wie beispielsweise Methyliodid in Gegenwart eines Phasentransferkatalysators, wie beispielsweise Tetrabutylammoniumbromid, in einem Zweiphasensystem, wie wässrige Natronlauge/Toluol, bei Temperaturen zwischen 10° C und 40° C umsetzt.

Die 4-Amino-Verbindungen der Formel (III) und deren Herstellung sind bereits beschrieben (vgl. hierzu die DE-A-32 40 308).

Bei dem erfindungsgemäßen Verfahren kommen als Verdünnungsmittel alle inerten organischen Lösungsmittel infrage. Hierzu gehören Kohlenwasserstoffe, wie Toluol, Xylol; chlorierte aromatische Kohlenwasserstoffe, wie Chlorbenzol, 1,2-Dichlorbenzol, 1,2,4-Trichlorbenzol; Ether, wie Tetrahydrofuran, Dioxan; Alkohole, wie Methanol, Ethanol, Propanol, Isopropanol; Amide, wie N,N-Dimethylformamid, Tetramethylharnstoff oder Sulfoxide, wie Dimethylsulfoxid. Vorzugsweise wird für die Umsetzung Isopropanol verwendet.

Die Reaktionstemperaturen können bei dem erfindungsgemäßen Verfahren in einem größeren Bereich variiert werden. Im allgemeinen arbeitet man bei Temperaturen zwischen 20° C und 170° C, vorzugsweise bei Temperaturen zwischen 60° C und 90° C.

Eine besonders vorteilhafte Ausführungsform des erfindungsgemäßen Verfahrens besteht darin, daß man in Gegenwart mindestens der äquimolaren Menge einer niederen aliphatischen Carbonsäure arbeitet. Vorzugsweise wird hierfür Essigsäure verwendet. Dieses Verfahren gestattet es mit relativ geringem Methylamin-Überschuß auszukommen. Bei dieser Ausführungsform kann die Reaktionsgeschwindigkeit durch Zusatz einer katalytischen Menge einer organischen Sulfonsäure erhöht werden. Vorzugsweise verwendet man hierfür p-Toluolsulfonsäure.

Bei der Durchführung des erfindungsgemäßen Verfahrens setzt man auf 1 Mol des 3-Alkylthio-triazinons der Formel (II) zweckmäßigerweise 1 bis 3 Mol einer niederen aliphatischen Carbonsäure und gegebenenfalls 0,01 bis 0,05 Mol einer organischen Sulfonsäure sowie 1 bis 7 Mol Methylamin ein, erhitzt bis zum Ende der Mercaptan-Abspaltung und arbeitet anschließend in üblicher Art und Weise auf.

Der erfindungsgemäße Wirkstoff kann als Defoliant, Desiccant, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob der erfindungsgemäße Stoff als totales oder selektives Herbizid wirkt, hängt im wesentlichen von der an gewendeten Menge ab.

Der erfindungsgemäße Wirkstoff kann z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung des erfindungsgemäßen Wirkstoffs ist jedoch keineswegs auf diese Gattungen beschränkt, sondern erstreckt sich in gleicher Weise auch auf andere Pflanzen.

Die Verbindung eignet sich in Abhängigkeit von der Konzentration zur Totalunkrautbekämpfung z.B. auf Industrie- und Gleisanlagen und auf Wegen und Plätzen mit und ohne Baumbewuchs. Ebenso kann die Verbindung zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei läßt sich der erfindungsgemäße Wirkstoff der Formel (I) mit besonders gutem Erfolg zur Bekämpfung dikotyler Unkräuter in Mais sowie auch in Getreide (Weizen, Gerste) einsetzen.

Der Wirkstoff kann in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen des Wirkstoffs mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolo-mit sowie synthetische Granulate aus anorganischen und organischen Mehlen sowie Granulate aus organischem Material wie Sägemehl, Kokosnußschalen, Maiskolben und Tabakstengeln; als Emulgier- und oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von

Eisen, Mangan, Bor, Kupfer, Kobalt, Molybdän und Zink verwendet werden.

Die Formulierungen enthalten jm allgemeinen zwischen 0,1 und 95 Gewichtsprozent Wirkstoff, vorzugsweise zwischen 0,5 und 90 %.

Der erfindungsgemäße Wirkstoff kann als solcher oder in seinen Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Auch Mischungen mit 2,4-Dichlorphenoxyessigsäure (2,4-D); 2,4-Dichlorphenoxypropionsäure (2,4-DP); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENZSULFURON); 3,5-Dibrom-4-hydroxy-benzonitril (BROMOXYNIL); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-Chlor-4-ethylamino-6-(3-cyanopropylamino)-1,3,5-triazin (CYANAZIN); 3,5-Diiod-4-hydroxybenzonitril (IOXYNIL); N,N-Dimethyl- N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); 2-{[[((4-Methoxy-6-methyl-1,3,5-triazin-2-yl)-amino)-carbonyl]-amino]-sulfonyl}-benzoesäure oder deren Methylester (METSULFURON); Methyl-2-{[(4,6-dimethyl-2-pyrimidinyl)-aminocarbonyl]-aminosulfonyl}-benzoat (SULFOMETURON); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); 2-tert-Butylamino-4-chlor-6-ethylamino-1,3,5-triazin (TERBUTHYLAZIN) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Der Wirkstoff kann als solcher, in Form seiner Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Der erfindungsgemäße Wirkstoff kann sowohl vor als auch nach dem Auflaufen der Pflanzen appliziert werden. Er kann auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung des erfindungsgemäßen Wirkstoffs geht aus den nachfolgenden Beispielen hervor.

Herstellungsbeispiel

Beispiel 1

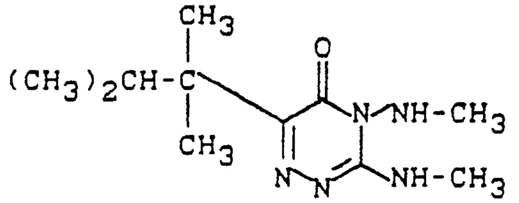

(I)

8,2 g (0,032 Mol) 6-(2,3-Dimethyl-2-butyl)-4-methyl-amino-3-methylthio-1,2,4-triazin-5(4H)-on werden bei Raumtemperatur zu einer Lösung aus 100 ml Isopropanol, 6 g (0,1 Mol) Eisessig und 6,2 g (0,2 Mol)

Methylamin gegeben. Man versetzt mit einer Spatelspitze p-Toluolsulfonsäure und erhitzt unter Rückfluß. Bei dieser Temperatur läßt man 48 Stunden nachrühren. Das Isopropanol wird im Vakuum abdestilliert, der Rückstand in Methylenchlorid aufgenommen, dreimal mit Wasser gewaschen und im Vakuum eingeengt. Nach Versetzen mit Diethylether und Anreiben kristallisiert der ölige Rückstand. Nach Absaugen und Trocknen erhält man 6,2 g (81,0 % der Theorie) 3,4-Bis-(methylamino)-6-(2,3-dimethyl-2-butyl)-1,2,4-triazin-5(4H)-on vom Schmelzpunkt 129-130 °C.

Herstellung der Vorprodukte

(a)

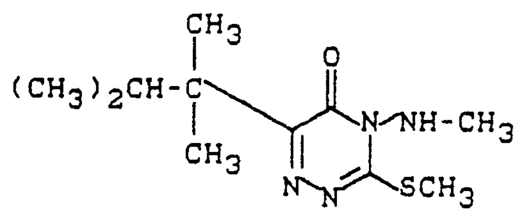

60,6 g (0,25 Mol) 4-Amino-6-(2,3-dimethyl-2-butyl)-3-methylthio-1,2,4-triazin-5(4H)-on werden bei Raumtemperatur in eine Mischung von 100 ml 45 %iger Natronlauge und 100 ml Toluol eingetragen. Bei Raumtemperatur gibt man anschließend 88,7 g (0,625 Mol) Methyliodid und 8,3 g Tetrabutylammoniumbromid auf einmal zu. Bei intensivem Rühren steigt die Temperatur auf 35 °C an. Man läßt das Reaktionsgemisch 30 Minuten lang ausrühren und trennt die organische Oberphase im Scheidetrichter ab. Die organische Phase wird in 1,5 l Eiswasser unter Rühren eingetragen und 1 Stunde intensiv verrührt. Der weiße, feinkristalline Niederschlag wird abgesaugt und gut mit Wasser nachgewaschen. Nach Trocknung bei 50 °C im Vakuum und Umkristallisation aus 80 ml Essigester erhält man 32,9 g (51,3 % der Theorie) 6-(2,3-Dimethyl-2-butyl)-4-methylamino-3-methylthio-1,2,4-triazin-5-(4H)-on vom Schmelzpunkt 117-118 °C.

(b) - vgl. DE-A-32 40 308:

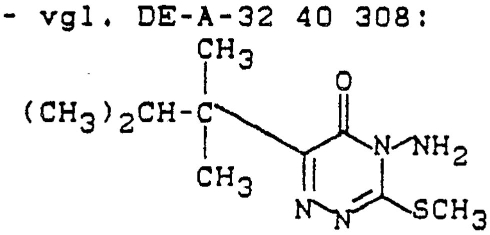

(III-1)

15,0 g (0,0697 Mol) 4-Amino-6-(2,3-dimethyl-2-butyl)-3-mercapto-1,2,4-triazin-5(4H)-on werden in 77 ml 1N Natronlauge gelöst und vom unlöslichen Rückstand abfiltriert. Das Filtrat wird bei Raumtemperatur mit 10,9 g (0,77 Mol) Methyliodid versetzt und 12 bis 15 Stunden gerührt. Zur Aufarbeitung saugt man den ausgefallenen Feststoff ab und wäscht ihn mehrfach mit Wasser. Die Trocknung erfolgt bei 40 °C bis 50 °C im Vakuum bis zur Gewichtskonstanz.

Man erhält 11,7 g (69,3 % der Theorie) an 4-Amino-6-(2,3-dimethyl-2-butyl)-3-methylthio-1,2,4-triazin-5-(4H)-on vom Schmelzpunkt 127 °C bis 128 °C.

(c)

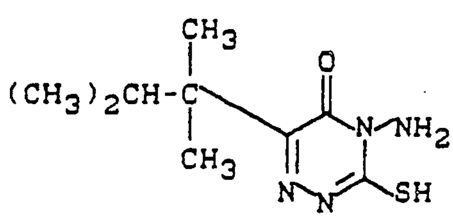

49 g (0,353 Mol) 3,3,4-Trimethyl-2-oxo-pentanoylnitril werden in 283 g einer 33 %igen Bromwasserstofflösung in Eisessig 4,5 Stunden bei 15 °C bis 20 °C gerührt. Danach fügt man tropfenweise unter Rühren und Eiskühlung eine Lösung von 41,2 g (0,388 Mol) Thiocarbohyrazid in 388 ml 1N Salzsäure zu, so daß

die Temperatur der Reaktionsmischung 20 °C nicht übersteigt. Nach beendeter Zugabe rührt man weitere 3 Tage bei Raumtemperatur. Danach wird das ausgefallene kristalline Produkt abgesaugt, mit Petrolether gewaschen und getrocknet.

Man erhält 26,3 g (35 % der Theorie) an 4-Amino-6-(2,3-dimethyl-2-butyl)-3-mercapto-1,2,4-triazin-5-(4H)-on vom Schmelzpunkt 266 °C bis 270 °C.

(d)

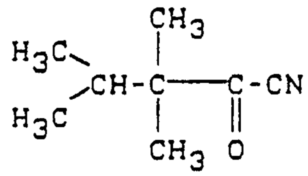

266 g (1,79 Mol) 2,2,3-Trimethylbutanoylchlorid werden auf 120 °C erwärmt und dann tropfenweise mit 177 g (1,79 Mol) Trimethylsilylcyanid versetzt, wobei die Mischung refluxiert. Während 16-stündigem Erhitzen destilliert freigesetztes Trimethylsilylchlorid langsam über einen Anschütz-Aufsatz ab und wird in einer Vorlage aufgefangen. Danach wird das Reaktionsprodukt im Wasserstrahlvakuum über eine Spiegelkolonne destilliert.

Man erhält 213,2 g (89 % der Theorie) an 3,3,4-Trimethyl-2-oxo-pentanoylnitril vom Siedepunkt 55 °C bis 56 °C/24 mbar.

(e)

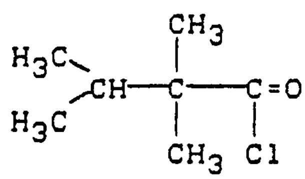

252 g (1,95 Mol) 2,2,3-Trimethylbuttersäure in 150 ml absolutem Chloroform werden bei Raumtemperatur tropfenweise unter Rühren mit 219 ml (2,96 Mol) Thionylchlorid versetzt und nach beendeter Zugabe weitere 5 Stunden bei Raumtemperatur gerührt. Zur Aufarbeitung destilliert man das Lösungsmittel ab und fraktioniert den Rückstand im Wasserstrahlvakuum.

Man erhält 266,6 g (92,2 % der Theorie) an 2,2,3-Trimethylbutanoylchlorid vom Siedepunkt 45 °C bis 48 °C/14 bis 18 mbar.

(f)

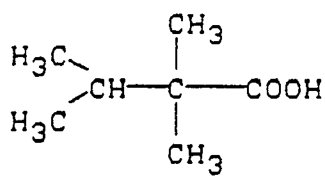

200 g (1,23 Mol) 1-Chlor-3,3,4-trimethyl-2-pentanon in 900 ml Dioxan werden unter Rühren und Eiskühlung bei 0 °C bis 5 °C zu einer Lösung von 492 g (12,3 Mol) Natriumhydroxid und 125,2 g (2,44 Mol) Brom in 2200 ml Wasser getropft. Man rührt die Mischung 16 Stunden bei Raumtemperatur. Vor der Aufarbeitung zerstört man überschüssiges Natriumhypobromit durch Zugabe von 122 g (0,64 Mol) Natriumpyrosulfit ($Na_2S_2O_5$). Nach 15-minütigem Rühren ist kein überschüssiges Hypobromit mehr nachweisbar.

Die wässrige Dioxanphase wird abgetrennt, mit konzenStrierter Salzsäure angesäuert und mit Essigester mehrfach extrahiert. Man wäscht die vereinigten organischen Phasen mit Wasser, trocknet über Natriumsulfat und entfernt das Lösungsmittel im Vakuum.

Man erhält 126,8 g (79,2 % der Theorie) an 2,2,3-Trimethylbuttersäure als Oel.

(g)

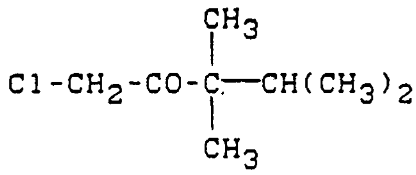

238 g (1 Mol) 1-Chlor-2-phenoxy-3,3,4-trimethyl-1-penten werden mit 500 g Ameisensäure und 50 g konzentrierter Salzsäure 2 Stunden bei 80 °C gerührt. Man verdünnt mit Methylenchlorid, wäscht einmal mit Wasser und viermal mit verdünnter Natronlauge. Nach dem Trocknen über Natriumsulfat wird das Lösungsmittel im Vakuum abgezogen und der Rückstand über eine Kolonne destilliert.

Man erhält 125 bis 135 g (77 bis 83 % der Theorie) 1-Chlor-3,3,4-trimethyl-2-pentanon vom Siedepunkt 88 °C bis 9 °C/16 mbar.

(h)

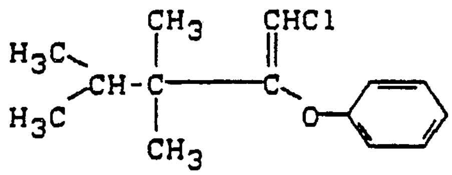

In 1,6 l ca. 100 °C heißes N-Methylpyrrolidon werden 487 g Natriumphenolat eingetragen und durch Erhitzen auf 200 °C gelöst. Im Verlauf von 3 Stunden werden 360 g (2 Mol) 1,1-Dichlor-3,3,4-trimethyl-1-penten so zugetropft, daß die Temperatur der Reaktionsmischung nicht unter 195 °C sinkt. Anschließend erhitzt man 8 Stunden auf 200 °C bis 210 °C. Zur Aufarbeitung wird die Lösung mit Methylenchlorid verdünnt und mit verdünnter Natronlauge ausgeschüttelt. Nach Trocknen und Abziehen des Lösungsmittels bleiben 406 g Rohprodukt zurück, das bei 105 °C bis 120 °C/0,1 mbar destilliert wird.

Man erhält 337 g (71 % der Theorie) 1-Chlor-2-phenoxy-3,3,4-trimethyl-1-penten.

(i)

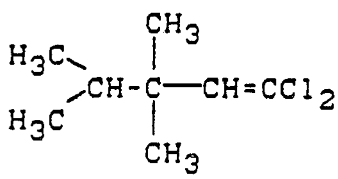

In eine trockene Apparatur mit Trockenrohr gibt man 20 g wasserfreies, gepulvertes Aluminiumchlorid portionsweise zu 2040 g (21 Mol) Vinylidenchlorid bei -10 °C. Dann tropft man im Temperaturbereich von 0 °C bis 10 °C 837 g (7 Mol) 2-Chlor-2,3-dimethylbutan zu. Man läßt auf 20 °C erwärmen und gibt weitere Aluminiumchlorid-Portionen (maximal 20 g) unter Kühlung zu, bis die Reaktion nicht mehr exotherm ist. Der Ansatz wird 4 Stunden bei Raumtemperatur nachgerührt; durch Eintropfen von 70 ml Essigsäure wird der Katalysator desaktiviert und die Mischung wird über Natriumsulfat filtriert. Durch Ein tropfdestillation bei 1 mbar werden die flüchtigen Bestandteile von den schwerflüchtigen getrennt. Das Destillat wird an einer Kolonne fraktioniert.

Man erhält 824 g bis 1226 g (65 bis 95 % der Thoerie) 1,1-Dichlor-3,3,4-trimethyl-1-penten vom Siedepunkt 60 °C bis 75 °C/20 mbar.

(j)

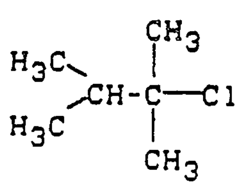

In 840 g (10 Mol) 2,3-Dimethyl-1-buten werden bei 10 °C bis 20 °C unter Eiskühlung ca. 440 g (12 Mol) Chlorwasserstoff aus der Bombe in 16 Stunden eingeleitet. Laut IR ist vollständiger Umsatz erfolgt. Überschüssiger Chlorwasserstoff wird in die Wasserstrahlpumpe gezogen.

Man erhält 1103 g (91 % der Theorie) 2-Chlor-2,3-dimethyl-butan.

Anwendungsbeispiel

In dem folgenden Anwendungsbeispiel wird die nachstehend aufgeführte Verbindung als Vergleichssubstanz eingesetzt:

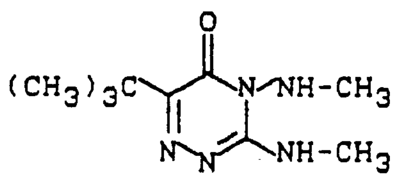

(A)

(bekannt aus EP-A-0 114 783)

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der Wirksamkeit ebenso wie in der Nutzpflanzenselektivität gegenüber dem Stand der Technik zeigt in diesem Test die Verbindung (I) gemäß Herstellungsbeispiel 1.

**Ansprüche**

1. 3,4-Bis-(methylamino)-6-(2,3-dimethyl-2-butyl)-1,2,4-triazin-5(4H)-on der Formel (I)

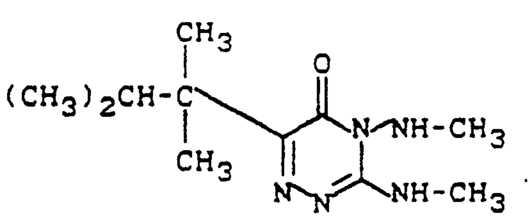

(I)

2. Verfahren zur Herstellung von 3,4-Bis-(methyl-amino)-6-(2,3-dimethyl-2-butyl)-1,2,4-triazin-5(4H)-on der Formel (I) gemäß Anspruch 1, dadurch gekennzeichnet, daß man ein 3- Alkylthio-4-methyl-amino-6-(2,3-dimethyl-2-butyl)-1,2,4-triazin-5(4H)-on der Formel (II)

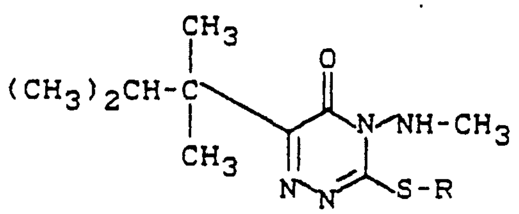

(II)

in welcher
R für Alkyl steht,
gegebenenfalls in Gegenwart eines Verdünnungsmittels und gegebenenfalls in Gegenwart einer niederen aliphatischen Carbonsäure mit Methylamin umsetzt.

3. Herbizide Mittel, gekennzeichnet durch einen Gehalt an 3,4-Bis-(methylamino)-6-(2,3-dimethyl-2-butyl)- 1,2,4-triazin-5(4H)-on der Formel (I) gemäß Anspruch 1.

4. Verfahren zur Bekämpfung von Unkraut, dadurch gekennzeichnet, daß man 3,4-Bis-(methylamino)-6-(2,3-dimethyl-2-butyl)-1,2,4-triazin-5(4H)-on der Formel (I) gemäß Anspruch 1 auf die Unkräuter oder ihren Lebensraum einwirken läßt.

5. Verwendung von 3,4-Bis-(methylamino)-6-(2,3-dimethyl-2-butyl)-1,2,4-triazin-5(4H)-on der Formel (I) gemäß Anspruch 1 zur Bekämpfung von unerwünschtem Pflanzenwachstum.

6. Verfahren zur Herstellung von herbiziden Mitteln, dadurch gekennzeichnet, daß man 3,4-Bis-(methylamino)-6-(2,3-dimethyl-2-butyl)-1,2,4-triazin-5(4H)-on der Formel (I) gemäß Anspruch 1 mit Streckmitteln und/oder oberflächenaktiven Mitteln vermischt.

7. 3-Alkylthio-4-methylamino-6-(2,3-dimethyl-2-butyl)-1,2,4-triazin-5(4H)-one der Formel (II)

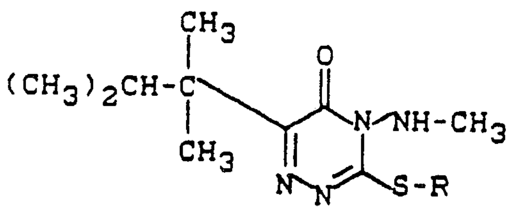

(II)

in welcher
R für Alkyl steht.

8. 6-(2,3-Dimethyl-2-butyl)-4-methylamino-3-methylthio-1,2,4-triazin-5(4H)-on der Formel (II-1)

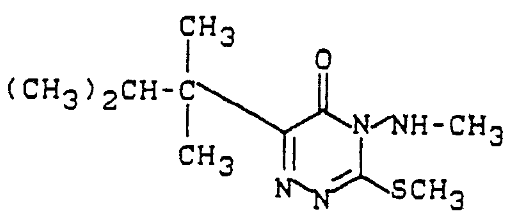

(II-1).

9. Verfahren zur Herstellung von 3-Alkylthio-4-methyl-amino-6-(2,3-dimethyl-2-butyl)-1,2,4-triazin-5(4H)-onen der Formel (II) gemäß Anspruch 7,

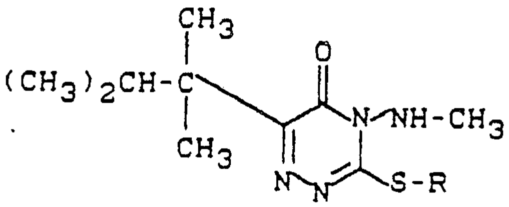

(II)

in welcher
R für Alkyl steht,

dadurch gekennzeichnet, daß man 4-Amino-Verbindungen der Formel (III),

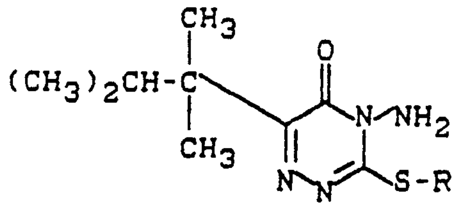

(III)

in welcher
R die oben angegebene Bedeutung hat,
mit einem Alkylierungsmittel in Gegenwart eines Phasentransferkatalysators in einem Zweiphasensystem bei Temperaturen zwischen 10 °C und 40 °C umsetzt.